# EUROPÄISCHE PATENTSCHRIFT

(11) **EP 0 580 956 B1**
(45) Veröffentlichungstag und Bekanntmachung des Hinweises auf die Patenterteilung: **06.03.1996**
(21) Anmeldenummer: 93105619.6
(22) Anmeldetag: 05.04.1993
(51) Int. Cl.: C07C 45/75, C07C 47/19, C07D 249/14

(54) **Verfahren zur katalytischen Herstellung von Kondensationsprodukten des Formaldehyds**
Process for the catalytic preparation of condensation products of formaldehyde
Procédé pour la préparation catalytique de produits de condensation de formaldéhyde

(30) Priorität: 11.04.1992 DE 4212264; 11.09.1992 DE 4230144
(43) Veröffentlichungstag der Anmeldung: 02.02.1994
(73) Patentinhaber: BASF Aktiengesellschaft, D-67063 Ludwigshafen (DE)
(72) Erfinder: Gehrer, Eugen, Dr., W-6700 Ludwigshafen (DE); Harder, Wolfgang, Dr., W-6940 Weinheim (DE); Ebel, Klaus, Dr., W-6700 Ludwigshafen (DE); Melder, Johann-Peter, Dr., W-6800 Mannheim 1 (DE); Teles, Joaquim Henrique, Dr., W-6700 Ludwigshafen (DE)

(56) Entgegenhaltungen:
- DE-C- 161 235
- US-A- 2 224 910
- US-A- 4 615 970
- TETRAHEDRON LETTERS, Bd. 21, 1980, Oxford, GB, Seiten 4517 - 4520; J. CASTELLS et al: "The 'Formoin Reaction': A promising entry to carbohydrates from formaldehyde"
- Cannon et al., Aust. J. Chem., 1980, 33, 2237-2247

## Beschreibung

Die vorliegende Erfindung betrifft ein Verfahren zur katalytischen Herstellung von Kondensationsprodukten des Formaldehyds.

Seit den Arbeiten von J. Castells (Tetrahedron Letters, 21, 4517-4520, (1980)) ist bekannt, daß man Thiazoliumylide als Katalysatoren für die katalytische Umpolung von Formaldehyd und somit für dessen Selbstkondensation zu Dihydroxyaceton verwenden kann und ebenso für die entsprechende Umsetzung von höheren Aldehyden zu Acyloinen.

Mit Thiazoliumyliden als Katalysatoren erhält man bei der Acyloinkondensation von Formaldehyd hauptsächlich Ketone, insbesondere Dihydroxyaceton (DHA). Aufgabe der vorliegenden Erfindung war es, ein Verfahren sowie neue Katalysatoren für die Kondensation von Formaldehyd zu finden, die bei dieser Reaktion ein anderes Produktspektrum liefern als Thiazoliumylide und die insbesondere die Herstellung der Kondensationsprodukte Glykolaldehyd und/oder Glycerinaldehyd in guten Selektivitäten ermöglichen sollten. Insbesondere Glykolaldehyd kann nicht mit Hilfe der Thiazoliumylid-Katalysatoren erhalten werden. Glykolaldehyd und Glycerinaldehyd sind wertvolle Zwischenprodukte zur Herstellung von Ethylenglykol, Glyoxal, Glycerin sowie zur Synthese von Wirkstoffen.

Dementsprechend wurde ein Verfahren zur katalytischen Herstellung von Kondensationsprodukten des Formaldehyds gefunden, das dadurch gekennzeichnet ist, daß man Formaldehyd oder Formaldehyd-liefernde Verbindungen in Gegenwart von mesoionischen Katalysatoren der Formel I 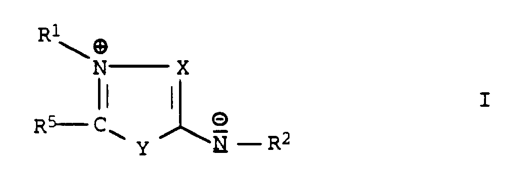 umsetzt, in der
- X: für ein Stickstoffatom oder eine Gruppe CR³ steht,
- Y: ein Schwefel- oder Selenatom oder eine Gruppe NR⁴ darstellt, und in der

die Reste R¹, R und R³ gleich oder verschieden sind und für aliphatische Gruppen mit 1 bis 30 Kohlenstoffatomen, für gegebenenfalls substituierte Arylgruppen, für gegebenenfalls substituierte Aralkylgruppen und/oder für gegebenenfalls substituierte Heteroarylgruppen stehen,
der Rest R⁴ für eine aliphatische Gruppe mit 1 bis 30 Kohlenstoffatomen, eine gegebenenfalls substituierte Arylgruppe, eine gegebenenfalls substituierte Aralkylgruppe oder eine gegebenenfalls substituierte Heteroarylgruppe steht, oder
in der der Rest R gemeinsam mit dem Rest R⁴ eine C₂- bis C₅-Alkylen- oder Alkenylen- oder eine C₆- bis C₁₄-Arylenbrücke bildet, und
in der der Rest R⁵ Wasserstoff, die Hydroxymethylengruppe -CH₂OH oder die Hydroxy-hydroxymethylen-methylidin-Gruppe -CH(OH) (CH₂OH) darstellt.

Bei den erfindungsgemäß verwendeten Katalysatoren handelt es sich somit um sogenannte mesoionische Verbindungen, also dipolare, fünfgliedrige Heterocyclen, die im gleichen Molekül je einen positiven und negativen Ladungsschwerpunkt haben, so daß sie nach außen neutral sind, obwohl für sie keine mesomere Grenzstruktur formuliert werden kann, in der nicht zugleich eine positive und negative elektrische Ladung vorhanden ist.

Die Reste R¹, R, R³ und R⁴ dienen im wesentlichen dazu, die mesoionischen Katalysatoren zu stabilisieren, d.h. durch ihre Anwesenheit dafür zu sorgen, daß sich die dipolaren Moleküle nicht in andere, nicht-mesoionische Moleküle, die keine getrennten positiven und negativen Ladungsschwerpunkte haben, umlagern können. Demgemäß können diese Reste eine Vielzahl von Bedeutungen haben.

So können R¹, R und R³ gleich oder verschieden sein und für aliphatische Gruppen mit 1 bis 30 Kohlenstoffatomen, wie C₁- bis C₃₀-Alkyl-, vorzugsweise C₁- bis C₁₀-Alkylgruppen, C₂- bis C₃₀, vorzugsweise C₂- bis C₁₀-Alkenyl- oder Alkinylgruppen mit 1 oder 2, vorzugsweise nur mit einer Mehrfachbindung, C₃- bis C₂₀-, vorzugsweise C₁- bis C₁₀-Cycloalkyl- oder -alkenylgruppen, C₃- bis C₂₀-Heterocycloalkylkyl- oder -alkenylgruppen, wie Piperidinyl-, Piperazinyl-, Pyrrolidinyl-, Imidazolidinyl-, Tetrahydrothienyl-, Tetrahydrofuranyl-, Tetrahydropyranyl-, Thiazolidinyl-, Oxazolidinyl-, Imidazolinyl-, Thiazolinyl-, Oxazolinyl- oder Kronenether-Gruppen, C₂- bis C₃₀-, vorzugsweise C₂- bis C₁₀-Alkoxygruppen mit einem oder mehreren, vorzugsweise nur mit einem Sauerstoffatom in der Etherkette, die an den Grundkörper des Katalysators I über ein Kohlenstoffatom gebunden sind, C₁- bis C₃₀-, vorzugsweise C₁- bis C₁₀-Fluor-, Chlor- und/ oder Brom-, vorzugsweise Fluor- und/oder Chlor-, insbesondere Fluor-enthaltende Halogenalkylgruppen mit einem oder mehreren, vorzugsweise mit 1 bis 3 Halogenatomen, im Falle von Fluoralkylgruppen vorteilhaft auch perfluorierte Fluoralkylgruppen, über ein Kohlenstoffatom an den Grundkörper des Katalysators I gebundene Aminogruppen, wie C₂- bis C₃₀-, vorzugsweise C₂- bis C₂₀-sekundäre Aminogruppen, C₃- bis C₃₀-, vorzugsweise C₃- bis C₂₁-tertiäre Aminogruppen, für gegebenenfalls substituierte Arylgruppen, vorzugsweise C₆- bis C₁₄-Arylgruppen, insbesondere für Phenyl-, Naphthyl-, Anthryl- oder Phenanthryl-Gruppen, für gegebenenfalls substituierte C₇- bis C₂₀-Aralkylgruppen, insbesondere die Benzyl-, Phenylethyl- oder Naphthylmethyl-Gruppe, für gegebenenfalls substituierte C₂- bis C₁₅-Heteroarylgruppen mit 1 bis 3 Stickstoffatomen oder einem Sauerstoff- oder Schwefelatom oder mit 1 bis 2 Stickstoffatomen und einem Sauerstoff- oder Schwefelatom im Ring, wie die Furanyl-, Thienyl-, Pyrrolyl-, Pyrazolyl-, Imidazolyl-, Thiazolyl-, Isothiazolyl-, Oxazolyl-, Isoxazolyl-, Pyridinyl-, Pyrimidinyl-, Pyrazinyl-, Chinolinyl-, Naphthyridinyl-, 1,2,4-Triazolyl- oder Acridinyl-Gruppe, stehen.

Sowohl die aliphatischen als auch die aromatischen und selbstverständlich auch die Aralkylreste können einfach oder mehrfach, vorzugsweise nicht mehr als dreifach mit Halogenatomen, Nitro-, Hydroxy-, Cyano-, Alkyl-, Alkoxy- oder unsubstituierten, einfach oder zweifach substituierten Aminogruppen substituiert sein. Da diese Substituenten in der Regel eine nur geringfügige Wirkung auf die katalytische Aktivität der betreffenden Katalysatoren I ausüben, werden hauptsächlich aufgrund ihrer kostengünstigeren Herstellung, die obengenannten unsubstituierten Reste R¹, R und R³ bevorzugt verwendet.

Der Rest R⁴ kann den Resten R¹, R und R³ gleich oder verschieden sein und außerdem noch gemeinsam mit dem Rest R eine C₂- bis C₅-Alkylen- oder Alkenylenbrücke oder eine C₆- bis C₁₄-Arylenbrücke, vorzugsweise eine o-Phenylen-, o-Naphthylen-, 1, 8-Naphthylen-, o-Fluorenylen-, 5,4-Fluorenylen-, o-Phenanthrylen-, 5,4-Phenanthrylen-, 9,10-Phenanthrylen-, 2,2'-Biphenylen-, o-Anthrylen- oder eine 1,9-Ahthrylenbrücke bilden.

Besonders bevorzugt werden Katalysatoren der Formel Ia 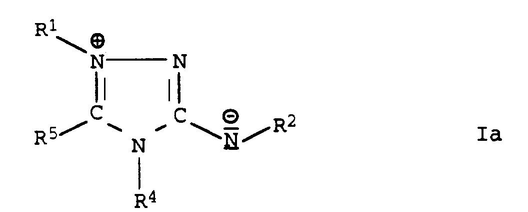 verwendet, in denen die Reste R¹, R, R⁴ und R⁵ die obengenannte Bedeutung haben. Von den Katalysatoren der Formel Ia werden wiederum diejenigen Verbindungen bevorzugt eingesetzt, in denen die Reste R¹, R und R⁴ gegebenenfalls substituierte Arylgruppen, insbesondere gegebenenfalls substituierte Phenylgruppen sind und R⁵ die obengenannte Bedeutung hat. Besonders bevorzugt ist im erfindungsgemäßen Verfahren die Verwendung von Nitron (IUPAC-Name: 1,4-Diphenyl-3-(phenylamino)-1H-1,2,4-triazoliumhydroxid, inneres Salz) der Formel Ib.

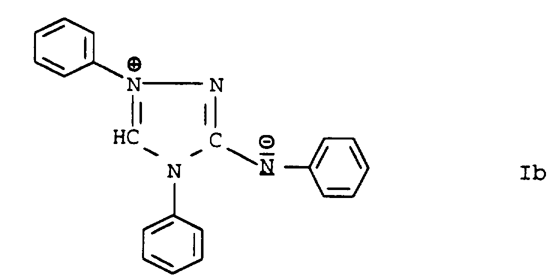

Im erfindungsgemäßen Verfahren kann der Formaldehyd in Form von Formaldehyd oder in Form von Formaldehyd-liefernden Verbindungen, z.B. als Paraformaldehyd, Formaldehydhalbacetallösungen, gasförmiger Formaldehyd oder als wäßrige Lösung (Formalin®) eingesetzt werden. Er wird im allgemeinen bei Temperaturen im Bereich von 20 - 160°C, bevorzugt bei 50 - 120°C und besonders bevorzugt bei 60 - 80°C, zusammen mit einem organischen Lösungsmittel und dem Katalysator zweckmäßigerweise 5 - 500 Minuten, bevorzugt 15 - 60 Minuten lang gerührt.

Der angewandte Druck ist im allgemeinen für das erfindungsgemäße Verfahren nicht kritisch. Es wird daher zweckmäßigerweise bei Atmosphärendruck oder unter dem Eigendruck des Reaktionssystems gearbeitet.

Für die Durchführung der Reaktion benötigt man in der Regel keine Hilfsbase. Da jedoch während der Reaktion als Nebenprodukte in geringem Ausmaß Säuren, z.B. Ameisensäure durch Cannizzaro-Reaktion, entstehen können und diese Säuren den Katalysator desaktivieren können, kann der Zusatz einer Base wie z.B. Triethylamin, Alkalimetall- oder Erdalkalimetallcarbonat, Alkalimetall- oder Erdalkalimetallhydroxid, Puffern wie Phosphatpuffer, Acetatpuffer etc. vorteilhaft sein, um solche Säuren abzupuffern. Dies kann insbesondere dann günstig sein, wenn man mit sehr kleinen Katalysatormengen arbeitet.

Die Verwendung einer Base ist folglich auch dann zweckmäßig, wenn der Katalysator in Form seiner katalytisch weniger aktiven Ahlagerungsverbindungen mit Carbonsäuren oder Mineralsäuren, also als Oniumsalz, in das erfindungsgemäße Verfahren eingebracht wird. In diesem Falle kann die an den Katalysator gebundene Säure durch den Zusatz einer nichtnucleophilen Base, z.B. durch den Zusatz von Formiaten oder Acetaten oder eines C₁- bis C₈-, vorzugsweise C₁- bis C₄-Alkylgruppen tragenden Trialkylamins oder Pyridinen, neutralisiert und der Katalysator aktiviert werden.

Das erfindungsgemäße Verfahren wird zweckmäßigerweise in Gegenwart eines Lösungsmittels durchgeführt. Als Lösungsmittel eignet sich grundsätzlich ein sehr breites Spektrum von Lösungsmitteln, wie Alkohole, z.B. Methanol, Ethanol, Propanol, Cyclohexanol, 2-Ethylhexanol und Hexadecylalkohol, Amide, z.B. Dimethylformamid (DMF), Dibutylformamid, Harnstoffe, wie Dimethylethylenharnstoff, Dimethylpropylenharnstoff, Carbonate, z.B. Propylencarbonat, Ethylencarbonat, aromatische Lösungsmittel, z.B. Toluol, Heterocyclen, z.B. Pyridin, N-Methylimidazol, N-Methylpyrrolidon, Ketone, z.B. Aceton, Ester, z.B. Essigsäureethylester, Ether, z.B. Methyl-tert.butylether, Diethylenglykolmethylether, Tetrahydrofuran, aromatische Nitroverbindungen, z.B. Nitrotoluol, tertiäre Amine, z.B. Triethylamin, halogenierte Kohlenwasserstoffe, wie Chlorbenzol oder Dichlorbenzol, Sulfide, wie Dimethylsulfoxid, Sulfone, wie Dimethylsulfon oder Sulfolan (Tetrahydrothiophen-1,1-dioxid), Trioxan und Nitrile, z.B. Acetonitril oder Propionitril. Die Menge des eingesetzten Lösungsmittel ist im allgemeinen nicht kritisch und von der Art des verwendeten Lösungsmittels abhängig, weshalb zweckmäßigerweise in einem Vorversuch die optimal einzusetzende Lösungsmittelmenge für das jeweils verwendete Lösungsmittel ermittelt wird.

Das Molverhältnis von Formaldehyd zu Katalysator kann in einem Bereich von 10 : 1 bis zu 10000 1 liegen. Bei Formaldehyd/Katalysatormolverhältnissen von mehr als 200 kann vorteilhaft eine Base oder ein Puffer zum Abfangen der Säuren zugesetzt werden.

Der Verwendung der erfindungsgemäß eingesetzten Katalysatoren der Formel I ist die Verwendung von deren synthetischen Vorläuferverbindungen gleichgestellt. So können diese Vorläuferverbindungen anstelle dieser Katalysatoren im erfindungsgemäßen Verfahren eingesetzt werden, da sich aus diesen Vorläuferverbindungen unter den üblicherweise im erfindungsgemäßen Verfahren angewandten Reaktionsbedingungen die entsprechenden mesoionischen Katalysatoren I in situ im Reaktionsgemisch bilden. Beispielsweise führt die Umsetzung von Triphenylaminoguanidin der Formel III mit Formaldehyd gemäß Gleichung (1) 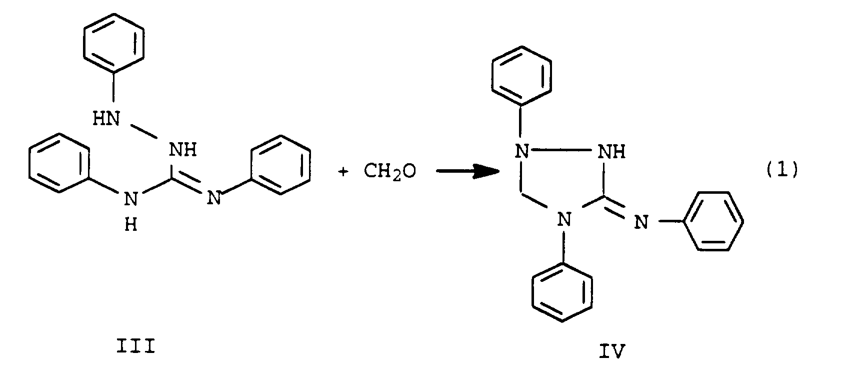 unter den im erfindungsgemäßen Verfahren angewandten Reaktionsbedingungen zur Bildung von Dihydronitron der Formel IV, welches wiederum leicht mit Hilfe von Oxidationsmitteln, z.B. Luftsauerstoff, leicht zu Nitron der Formel Ib oxidiert, das die eigentliche katalytisch aktive Verbindung ist. Die gleiche Wirkung wird erzielt, wenn das Guanidin III in der Reaktionsmischung mit Ameisensäure umgesetzt wird. Dabei bildet sich sogleich das Nitron Ib, ohne daß die Zwischenstufe IV durchlaufen wird. Diese beiden Verfahrensweisen können auch zur in situ-Erzeugung der übrigen erfindungsgemäß verwendbaren mesoionischen Verbindungen, insbesondere von denen der Formel Ia, aus den entsprechend substituierten Aminoguanidinverbindungen der Formel II 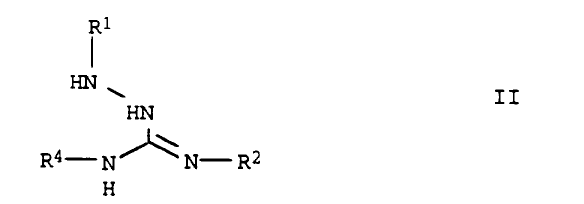 benutzt werden, in der die Reste R¹, R und R⁴ die zuvor genannte Bedeutung haben. Wird der Aminoguanidin II und Formaldehyd enthaltenden Reaktionsmischung Ameisensäure zur in situ-Erzeugung der katalytisch aktiven Verbindungen I zugesetzt, ist entsprechend den obigen Darlegungen die Anwesenheit eines Oxidationsmittels nicht erforderlich.

Man kann die Reaktion sowohl in homogener Phase, als auch in Suspension oder auch in einem flüssigen Zweiphasensystem durchführen. Im letztgenannten Fall wird z.B. als Rohstoff wässeriger Formaldehyd eingesetzt und der Katalysator in einem organischen, mit Wasser nicht mischbaren Lösungsmittel, vorzugsweise in einem langkettigen, insbesondere in einem C₆- bis C₂₀-Alkohol, gelöst. Unter diesen Phasentransferbedingungen wird dabei der Formaldehyd in die organische Phase extrahiert, dort am Katalysator umgesetzt und das hydrophile Produkt (Glykolaldehyd, Glycerinaldehyd) in die wässerige Phase zurückextrahiert.

Mit dem erfindungsgemäßen Verfahren lassen sich Kondensationsprodukte des Formaldehyds, insbesondere Glykolaldehyd, Glycerinaldehyd und C₄-Zucker herstellen, wobei die Wahl des Katalysators I steuern läßt. Der chemische Mechanismus, der dieser Umsetzung zugrundeliegt ist noch nicht vollständig aufgeklärt. Es wird jedoch vermutet, daß das katalytische aktive Zentrum der erfindungsgemäßen Katalysatoren sich an der 5-Position der mesoionischen Katalysatoren I befindet. Anlaß zu dieser Annahme gibt der Umstand, daß bei der Umsetzung der Katalysatoren I mit Formaldehyd, Katalysator-Formaldehyd-Addukte isoliert werden können, nämlich solche, in denen R⁵ eine -CH₂OH oder eine -CH(OH) (CH₂OH)-Gruppe ist, die selbst ebenfalls katalytisch aktiv sind. Es ist sehr überraschend, daß sich mit den erfindungsgemäßen Katalysatoren hauptsächlich die obengenannten Formaldehyd-Kondensationsprodukte bilden, wohingegen Dihydroxyaceton nur in geringen Mengen entsteht. Dieses katalytische Verhalten steht in klarem Gegensatz zum Verhalten von Thiazoliumylid-Katalysatoren, die bei der Kondensation von Formaldehyd hauptsächlich Dihydroxyaceton, indes keinen Glykolaldehyd liefern.

Nitron ist im Handel erhältlich oder kann nach Busch und Mehrtens, Ber. 38, 4049 (1905) durch die Umsetzung von Triphenylaminoguanidin mit Ameisensäure hergestellt werden. Auf analoge Weise können auch die anderen erfindungsgemäß verwendbaren Nitronderivate aus entsprechend substituierten Aminoguanidinen und Ameisensäure erzeugt werden.

### Beispiele

### Beispiel 1

Ein Ansatz aus 4.0 g (133 mmol) Paraformaldehyd, x g (s. Tabelle) Nitron, 15.8 g Ethylacetat und 0.2 g Molekularsieb wurde 15 Minuten lang bei 80°C in einem gläsernen Druckgefäß gerührt. Die Reaktionsausträge lieferten folgende Ergebnisse:

| Menge Nitron [g] | Molverhältnis Formaldehyd/Nitron | Umsatz [%] | Selektivität in % | |
|---|---|---|---|---|
| | | | Glykolaldehyd | Glycerinaldehyd |
| 0,201 | 207 | 29,4 | 86,2 | 10,1 |
| 0,103 | 405 | 19,2 | 90,1 | 7,0 |
| 0,051 | 816 | 12,6 | 91,0 | 5,4 |
| 0,025 | 1695 | 6,2 | 94,3 | 2,8 |

### Beispiel 2

Ein Ansatz aus 3.9 g (130 mmol) Paraformaldehyd, 0.26 g (0.87 mmol) Nitron, 15.8 g Ethylacetat und 0.2 g Molekularsieb wurde 60 Minuten lang bei 80°C in einem gläsernen Druckgefäß gerührt. Nach je 15, 30 und 60 Minuten wurden Proben gezogen und analysiert.

Ausbeuten in % bezogen auf den eingesetzten Formaldehyd

| Reaktionszeit | Glykolaldehyd | Glycerinaldehyd | Dihydroxyaceton |
|---|---|---|---|
| 15 Min | 36,0 % | 15,2 % | 0,3 % |
| 30 Min | 44,8 % | 24,9 % | 0,5 % |
| 60 Min | 45,8 % | 35,0 % | 1,1 % |

### Beispiel 3

Eine zweiphasige Mischung aus 4.83 g (60 mmol) Formalin® (37%ig), 5 g Chloroform und 0.47 g (1.52 mmol) Nitron wurde im gläsernen Druckgefäß 60 Minuten lang bei 80°C intensiv gerührt. Nach 15, 30 und 60 Minuten wurden Proben gezogen und analysiert.

Ausbeuten in % bezogen auf den eingesetzten Formaldehyd

| Reaktionszeit | Glykolaldehyd | Glycerinaldehyd |
|---|---|---|
| 15 Min | 23,9 % | 15,3 % |
| 30 Min | 23,8 % | 15,7 % |
| 60 Min | 23,0 % | 14,0 % |

### Beispiel 4

Dieses Beispiel belegt, daß ein breites Spektrum von Lösungsmitteln in Frage kommt und besonders mäßig polare Lösungsmittel (Chloroform, Tetrahydrofuran, Pyridin) für eine hohe Glykolaldehyd-Selektivität bevorzugt werden.

Ein Ansatz aus 2 g (66.7 mmol) Paraformaldehyd, 0.1 g (0.33 mmol) Nitron, 7.9 g Lösungsmittel und 0.1 g Molsieb wurde 30 Minuten lang bei 80°C gerührt.

### Beispiel 4:

Ausbeuten in % bezogen auf den eingesetzten Formaldehyd

| Lösungsmittel | Glycolaldehyd | Glycerinaldehyd | höhere Zucker (C4+C5) |
|---|---|---|---|
| Methanol | 5.6 | 6.8 | 16.9 |
| Ethanol | 12.7 | 7.5 | 3.3 |
| n-Propanol | 15.4 | 11.0 | 5.1 |
| i-Propanol | 21.8 | 14.0 | 4.9 |
| t-Butanol | 36.9 | 12.9 | 2.7 |
| Cyclohexanol | 20.8 | 12.4 | 4.5 |
| 2-Ethylhexanol | 24.1 | 20.9 | 9.5 |
| Hexadecanol | 8.6 | 5.4 | 3.8 |
| DMF | 23.5 | 4.6 | 0.4 |
| Dibutylformamid | 0.6 | 0.1 | 4.5 |
| Dimethylethylencarbonat | 8.4 | 1.9 | 7.4 |
| Toluol | 6.1 | 6.5 | 6.7 |
| Pyridin | 46.4 | 8.2 | 1.7 |
| N-Methylimidazol | 18.1 | 10.7 | 4.7 |
| N-Methylpyrrolidon | 7.0 | 0.6 | 0.7 |
| Wasser | 0.6 | 0.6 | 0.8 |
| Aceton | 32.0 | 5.5 | 0.5 |
| Essigsäureethylester | 59.4 | 10.9 | 2.2 |
| Methyl-tert.-butylether | 0.3 | 0.3 | 0.0 |
| Diethylenglykoldimethylether | 41.6 | 5.0 | 0.2 |
| Tetrahydrofuran | 56.6 | 14.1 | 3.4 |
| Nitrotoluol | 15.8 | 4.5 | 0.8 |
| Nitroethan | 0.8 | 0.4 | 0.2 |
| Acetonitril | 15.8 | 2.9 | 0.2 |
| Chloroform | 53.3 | 21.4 | 7.7 |
| Propylenglycol | 4.9 | 9.5 | 18.6 |
| Triethylamin | 1.9 | 3.7 | 17.6 |
| Trioxan | 27.5 | 6.4 | 1.6 |

### Beispiel 5

Diese Beispiele belegen, daß man die Reaktion in einem weiten Temperaturbereich durchführen kann.

Es wurde jeweils eine Suspension von 20 Gew.-% Paraformaldehyd in DMF zusammen mit dem Katalysator Nitron bei der angegebenen Temperatur und der angegebenen Reaktionszeit gerührt.

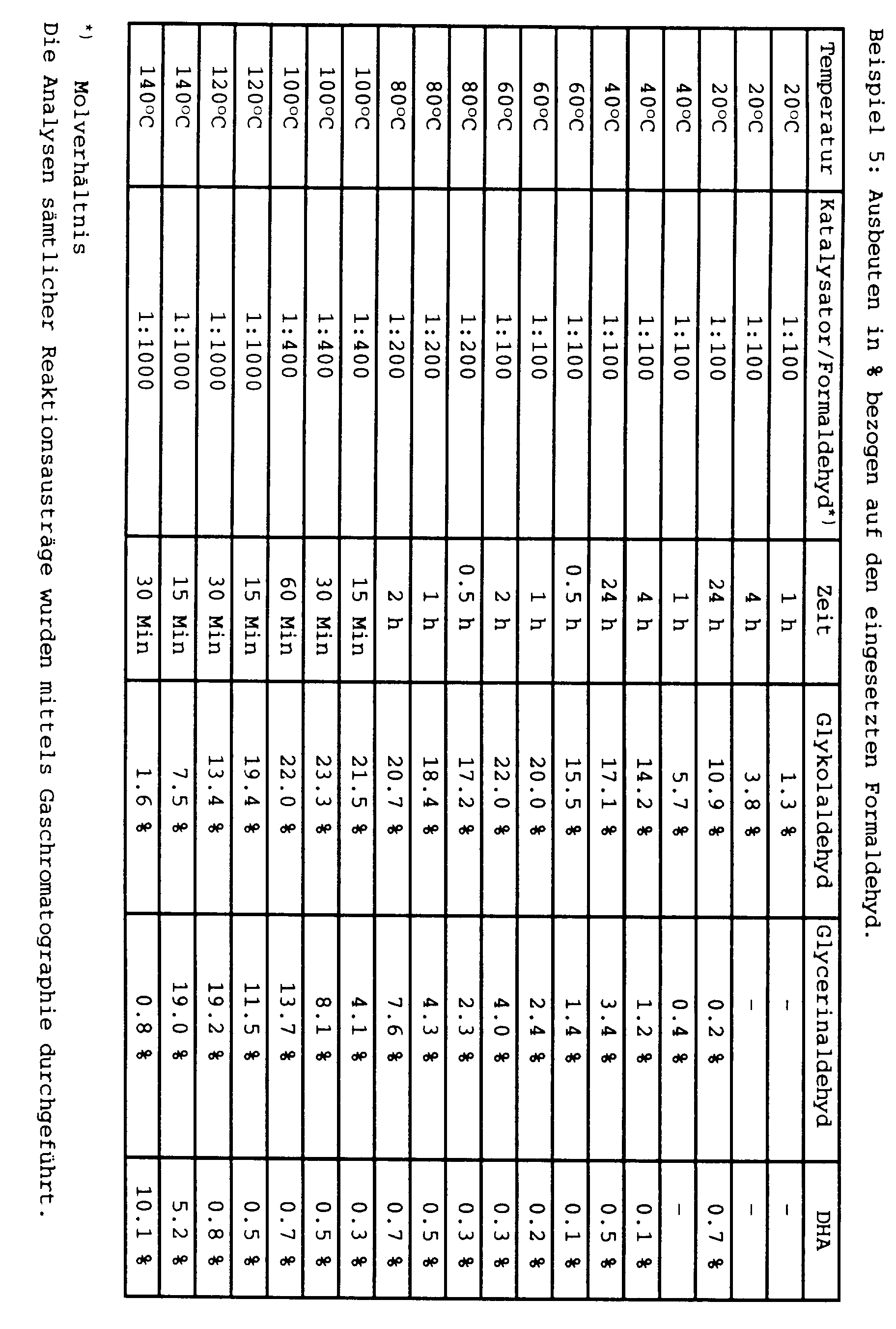

### Herstellung der Katalysatoren

### Triphenylaminoguanidin II

100 g (0,44 mol) N,N'-Diphenylthioharnstoff wurden in 300 ml Toluol suspendiert und zusammen mit 100 g (0,45 mol) PbO 15 Minuten bei 80°C gerührt (bis sich das tiefschwarze PbS vollständig abgeschieden hat). Die heiße Lösung wurde filtriert und das Filtrat mit 50 g (0,46 mol) Phenylhydrazin versetzt. Diese Lösung wurde 30 Minuten bei 80°C gerührt. Nach Abkühlung kristallisierte das Produkt aus.

### 1,4-Di-(2-methylphenyl)-3-(2-methylphenylamino)-1H-1,2,4-triazoliumhydroxid, inneres Salz VI

1,4 g (3,67 mmol) Tri-(2-methylphenyl)-aminoguanidin - hergestellt nach Busch, Ber. 38, 856 (1905) durch die Umsetzung von 2-Methylphenylhydrazin mit Di-(2-methylphenyl)-carbodiimid (hergestellt nach: Campbell et al.: J. Am. Chem. Soc. 84, 3673 (1952)) - wurden 20 Stunden mit 7 ml konzentrierter Ameisensäure in einem gläsernen Druckgefäß bei 140°C gerührt. Nach dem Abkühlen wurde die Reaktionsmischung mit Wasser verdünnt, mit Ammoniak neutralisiert und mit Chloroform extrahiert. Die organische Phase wurde über Natriumsulfat getrocknet, das Lösungsmittel wurde abdestilliert und der Rückstand an Kieselgel zuerst mit Diethylether, dann mit Methanol als Eluens chromatographiert.
Schmelzpunkt: 160 - 165°C
Massenspektrum: Molpeak: 354 m/e

Analog wurde durch die Umsetzung von Phenylhydrazin mit Di-(2-methylphenyl)-carbodiimid und die anschließende Cyclisierung des erhaltenen Kondensationsproduktes mit Ameisensäure 1-Phenyl-4-(2-methylphenyl)-3-(2-methylphenylamino)-1H-1,2,4-triazoliumhydroxid, inneres Salz V erhalten.

### Naphthonitron-Hydrojodid (9-Phenyl-7H-[1,2,4]-triazolo-[4,3-a]-perimidiniumjodid) VII

Zu einer Suspension von 19,2 g (56 mmol) 2-(Methylthio)perimidiniumjodid, das nach Herbert et al, J. Med. Chem. 30, 2081 (1987) hergestellt worden war, in 120 ml Methanol wurden den 28,1 ml (286 mmol) Phenylhydrazin gegeben und das erhaltene Gemisch 2 h unter Rückfluß gekocht. Nach beendeter Reaktion wurde das Lösungsmittel entfernt und der harzige Rückstand 2 h mit 30 ml Methanol digeriert. Der hierbei ausfallende Feststoff wurde abfiltriert, mit wenig kaltem Methanol gewaschen und im Exikkator getrocknet. Nach den Ergebnissen der NMR-spektroskopischen und massenspektrometrischen Untersuchungen sowie der Elementaranalyse handelt es sich bei dem erhaltenen Feststoff um N-Phenyl-N'-(2-perimidinium)-hydrazin-jodid. 2,0 g (5,0 mmol) dieser Verbindung wurden mit 10 ml konzentrierter Ameisensäure in einem 25 ml Glasautoklaven 2 Stunden bei 175°C gerührt. Nach dem Abkühlen wurde die Lösung mit 130 ml Wasser vermischt und der dabei ausfallende Feststoff abfiltriert und aus Ethanol umkristallisiert.
Massenspektrum: Molpeak 285 m/e
Schmelzpunkt: > 300°C
Korrekte Elementaranalyse

### 1-Phenyl-3-cyclohexylamino-4-cyclohexyl-1H-1,2,4-triazoliumformiat VIII

Zu 20,0 g (98 mmol) N,N'-Dicyclohexylcarbodiimid (erhältlich nach dem Verfahren von J. Am. Chem. Soc. 84, 3673 (1962) aus Cyclohexylisocyanat) in 900 ml Toluol wurden bei 40°C 10,6 g (98 mmol) Phenylhydrazin gegeben. Es wurde noch 2 h Raumtemperatur gerührt und anschließend das Lösungsmittel bei vermindertem Druck abdestilliert. Der Rückstand wurde mit 4,5 g Ameisensäure und 100 ml Diethylether vermischt, wobei es zur Ausfällung eines Feststoffs kam. Nach Abtrennung des Ethers wurden zum Feststoff 54,8 g Ameisensäure gegeben und 20 h auf 112°C erhitzt. Das Reaktionsgemisch wurde auf Raumtemperatur abgekühlt, mit 500 ml Wasser vermengt, unlösliche Feststoffpartikel abfiltriert und die erhaltene Lösung mit wäßriger Ammoniaklösung schwach basisch gestellt. Der sich dabei bildende Niederschlag wurde abfiltriert und in Dichlormethan gelöst. Nach Trocknung über Natriumsulfat und Entfernung des Lösungsmittels wurde der Feststoff aus Ethanol umkristallisiert.
Massenspektrum: Molpeak 325 m/e
Schmelzpunkt: 178°C

### Standardtest für Katalysatoraktivität

Die zuvor beschriebenen mesoionischen Verbindungen wurden, wie folgt, auf ihre katalytische Aktivität im erfindungsgemäßen Verfahren untersucht.

2,0 g (66,7 mmol) Paraformaldehyd wurden in 18 g Dimethylformamid suspendiert. Zu dieser Suspension wurden 0,33 mmol des mesoionischen Katalysators gegeben (Formaldehyd/Katalysator-Molverhältnis = 200:1) und dieser Ansatz 1 h bei 80°C gerührt. Wurden die Katalysatoren in Form ihrer Oniumsalze eingesetzt (Katalysatoren VII und VIII), so wurde daraus der aktive Katalysator durch Zugabe von 1 Äquivalent Triethylamin freigesetzt.

Die Reaktionsprodukte wurden folgendermaßen analysiert: 0,5 g Reaktionsprodukt wurden mit 10 g Oximierungsreagenz (5,0 g Butandiol-1,4 als innerer Standard für die Gaschromatographie und 70 g Hydroxylaminhydrochlorid in 1000 g Pyridin) vermischt und eine Stunde auf 70°C erwärmt. 1 ml dieser Lösung wurde dann mit 1 ml Hexamethyldisilazan und 1 ml Trimethylsilylchlorid vermischt und 0,5 bis 1 h bei Raumtemperatur stehengelassen bis Pyridiniumchlorid in großen Flocken ausfiel. Die Mischung wurde filtriert und die erhaltene Lösung direkt für die gaschromatographische Bestimmung der Reaktionsprodukte verwendet.

Die mit den einzelnen Katalysatoren erzielten Ergebnisse sind in der nachfolgenden Tabelle aufgelistet. In dieser Tabelle werden die Abkürzungen mit den folgenden Bedeutungen verwendet: GA - Glykolaldehyd; GlyAld - Glycerinaldehyd; DHA - Dihydroxyaceton; C₄ - Mischung aus C₄-Kohlenhydraten (Tetrosen); Et₃N - Triethylamin.

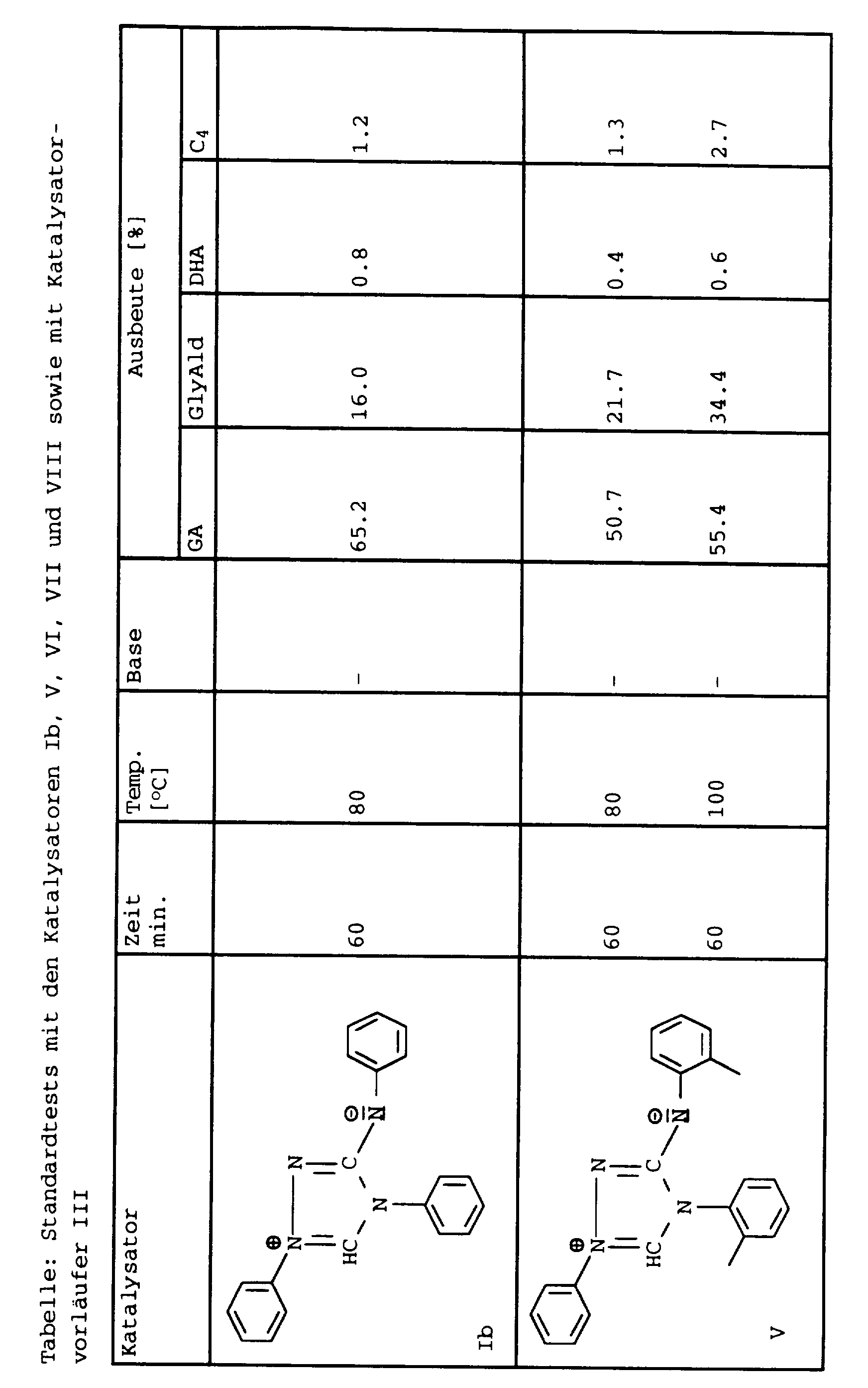

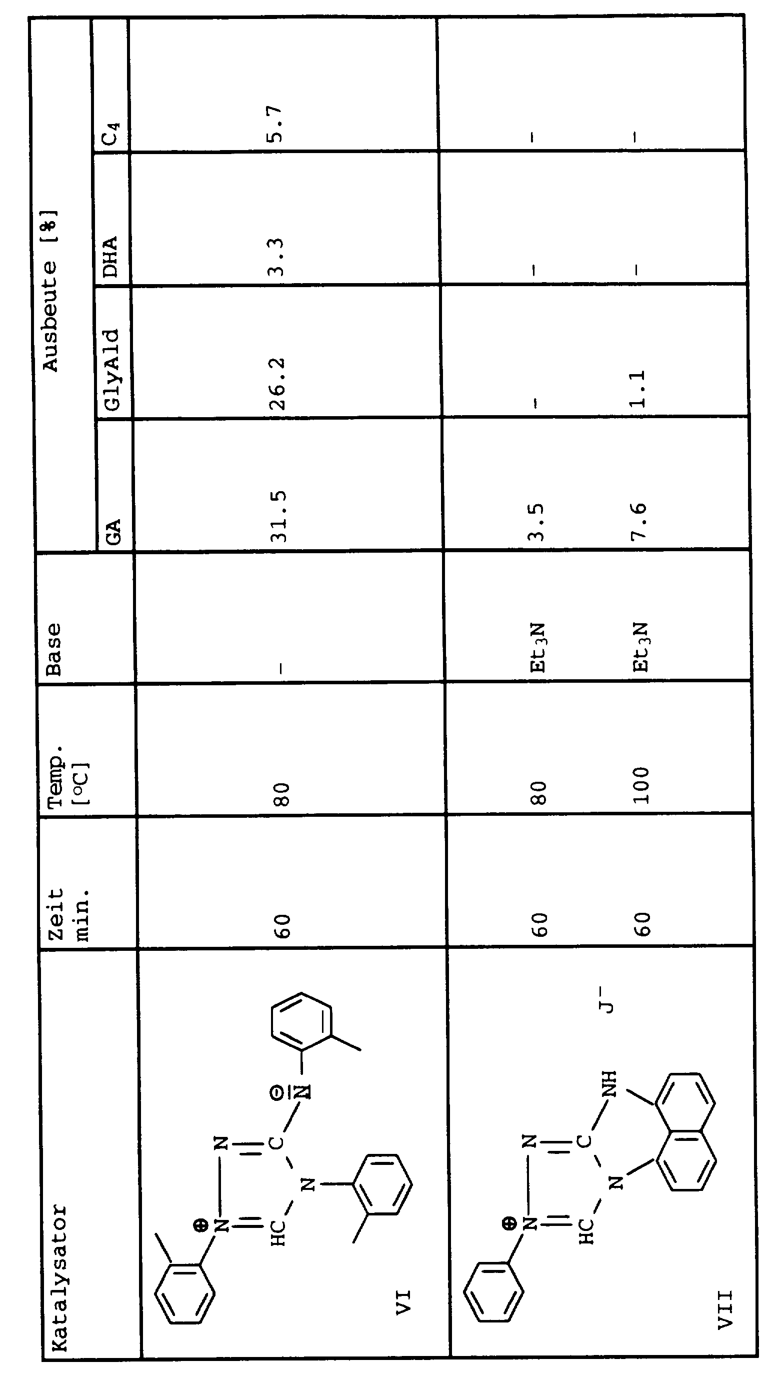

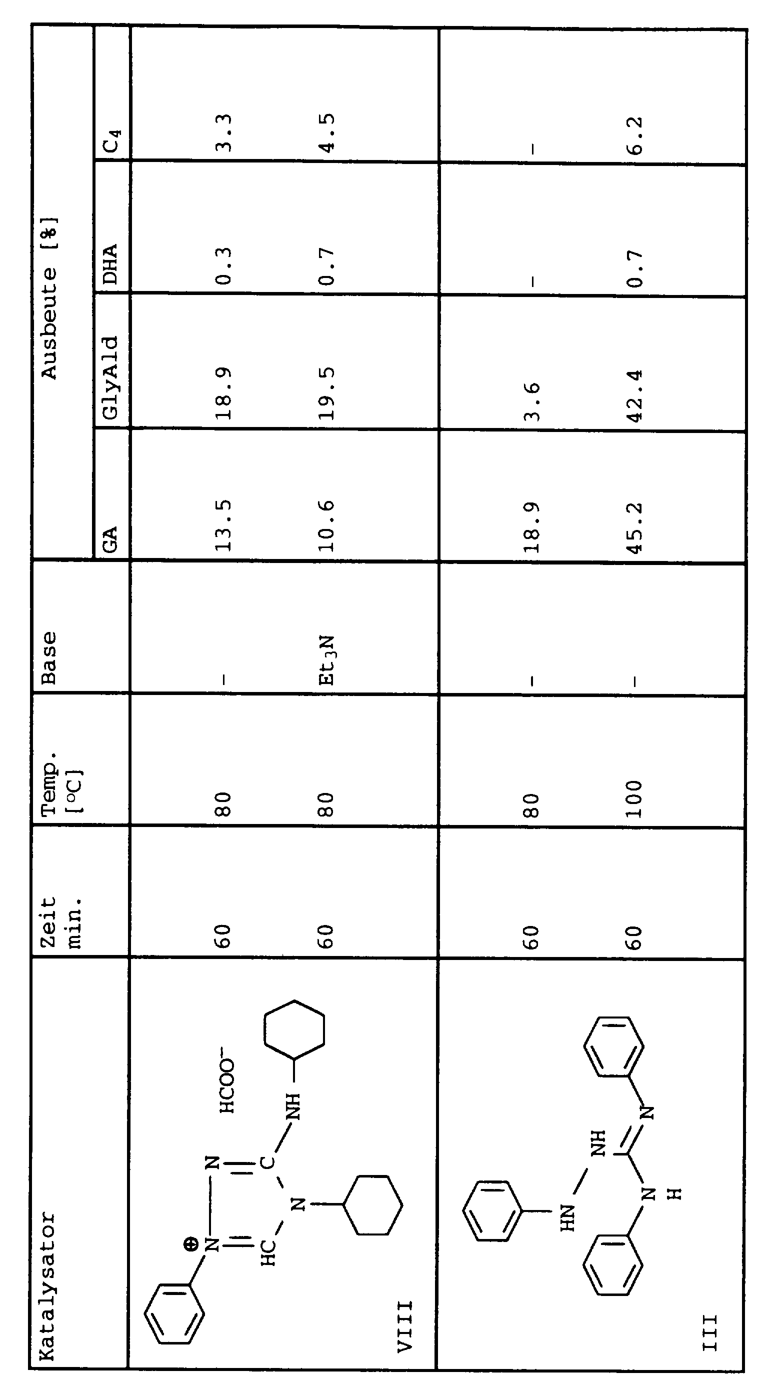

## Patentansprüche

1. Verfahren zur katalytischen Herstellung von Kondensationsprodukten des Formaldehyds, dadurch gekennzeichnet, daß man Formaldehyd oder Formaldehyd-liefernde Verbindungen in Gegenwart von mesoionischen Katalysatoren der Formel I 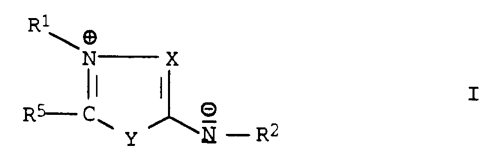 umsetzt, in der
X für ein Stickstoffatom oder eine Gruppe CR³ steht,
Y ein Schwefel- oder Selenatom oder eine Gruppe NR⁴ darstellt, und in der
die Reste R¹, R und R³ gleich oder verschieden sind und für aliphatische Gruppen mit 1 bis 30 Kohlenstoffatomen, für gegebenenfalls substituierte Arylgruppen, für gegebenenfalls substituierte Aralkylgruppen und/oder für gegebenenfalls substituierte Heteroarylgruppen stehen,
der Rest R⁴ für eine aliphatische Gruppe mit 1 bis 30 Kohlenstoffatomen, eine gegebenenfalls substituierte Arylgruppe, eine gegebenenfalls substituierte Aralkylgruppe oder eine gegebenenfalls substituierte Heteroarylgruppe steht, oder
in der der Rest R gemeinsam mit dem Rest R⁴ eine C₂- bis C₅-Alkylen- oder Alkenylen- oder eine C₆- bis C₁₄-Arylenbrücke bildet, und
in der der Rest R⁵ Wasserstoff, die Hydroxymethylengruppe -CH₂OH oder die Hydroxy-hydroxymethylen-methylidin-Gruppe -CH(OH)(CH₂OH) darstellt.

2. Verfahren nach Anspruch 1, dadurch gekennzeichnet, daß man Formaldehyd oder Formaldehyd-liefernde Verbindungen in Gegenwart von mesoionischen Katalysatoren der Formel I 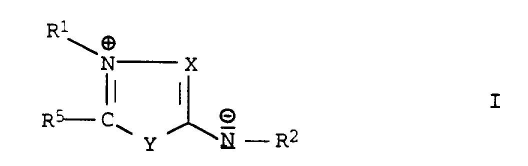 umsetzt, in der X, Y und R⁵ die in Anspruch 1 genannte Bedeutung haben und in der
die Reste R¹, R und R³ gleich oder verschieden sind und für C₁- bis C₃₀-Alkylgruppen, C₂- bis C₃₀-Alkenyl- oder Alkinylgruppen mit 1 oder 2, Mehrfachbindungen, C₃- bis C₂₀-Cycloalkyl- oder -alkenylgruppen, C₃- bis C₂₀-Heterocycloalkyl- oder -alkenylgruppen, über ein Kohlenstoffatom an den Grundkörper des Katalysators I gebundene C₂- bis C₃₀-Alkoxygruppen mit einem oder mehreren Sauerstoffatomen in der Etherkette, C₁- bis C₃₀-Fluor-, Chlor- und/oder Brom- enthaltende Halogenalkylgruppen mit einem oder mehreren Halogenatomen, über ein Kohlenstoffatom an den Grundkörper des Katalysators I gebundene C₂- bis C₃₀-sekundäre Aminogruppen oder C₃- bis C₃₀-tertiäre Aminogruppen, für gegebenenfalls substituierte C₆- bis C₁₄-Arylgruppen, für gegebenenfalls substituierte C₇- bis C₂₀-Aralkylgruppen und/oder für gegebenenfalls substituierte C₂- bis C₁₅-Heteroarylgruppen mit 1 bis 3 Stickstoffatomen oder einem Sauerstoff oder Schwefelatom oder mit 1 bis 2 Stickstoffatomen und einem Sauerstoff- oder Schwefelatom im Ring, stehen, und
der Rest R⁴ den Resten R¹, R oder R³ gleich oder verschieden ist oder gemeinsam mit dem Rest R eine C₂- bis C₅-Alkylen- oder Alkenylen- oder eine C₆- bis C₁₄-Arylenbrücke bildet.

3. Verfahren gemäß Anspruch 1, dadurch gekennzeichnet, daß man als Katalysator eine mesoionische Triazoliumverbindung der Formel Ia 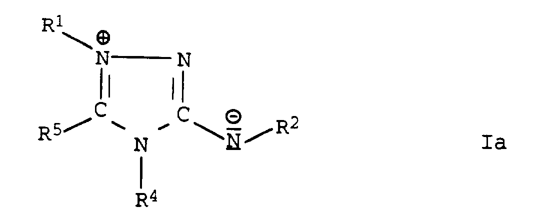 verwendet, in der R¹, R, R⁵ und R⁴ die in Anspruch 1 genannte Bedeutung haben.

4. Verfahren gemäß Anspruch 1, dadurch gekennzeichnet, daß man als Katalysator Nitron der Formel Ib 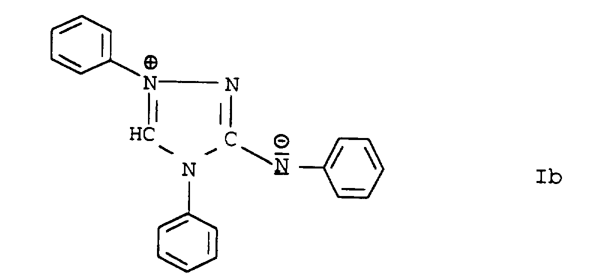 verwendet.

5. Verfahren gemäß Anspruch 3, dadurch gekennzeichnet, daß man die Katalysatoren der Formel Ia, worin R⁵ Wasserstoff ist, in situ im Reaktionsgemisch aus Aminoguanidinverbindungen der Formel II 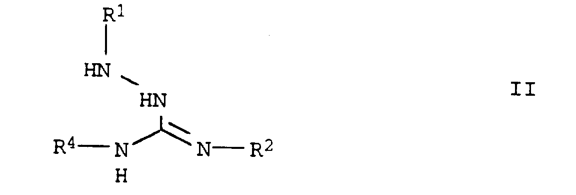 in der R¹, R und R⁴ die in Anspruch 1 genannte Bedeutung haben, und Ameisensäure oder aus den Aminoguanidinverbindungen II und Formaldehyd in Gegenwart eines Oxidationsmittels erzeugt.

6. Verfahren nach Anspruch 1, dadurch gekennzeichnet, daß man die mesoionischen Katalysatoren I in Form ihrer Oniumsalze mit Carbonsäuren oder Mineralsäuren einsetzt und daraus die Katalysatoren I mit Hilfe einer Base freisetzt.

## Claims

1. A process for catalytic preparation of condensation products of formaldehyde, characterized in that formaldehyde or formaldehyde-affording compounds are reacted in the presence of mesoionic catalysts of the formula I 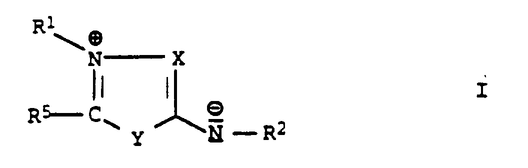 where
X is a nitrogen atom or a group CR³, and
Y is a sulfur or selenium atom or a group NR⁴, and where
the radicals R¹, R and R³ are identical or different and represent aliphatic groups having 1 to 30 carbon atoms, optionally substituted aryl groups, optionally substituted aralkyl groups and/or optionally substituted hetaryl groups,
the radical R⁴ is an aliphatic group having 1 to 30 carbon atoms, an optionally substituted aryl group, an optionally substituted aralkyl group or an optionally substituted hetaryl group, or
where the radical R combines with the radical R⁴ to form a C₂- to C₅alkylene or alkenylene or a C₆- to C₁₄-arylene bridge, and
where the radical R⁵ is hydrogen, the hydroxymethylene group -CH₂OH or the hydroxyhydroxymethylenemethylidene group -CH(OH) (CR₂OH).

2. A process as claimed in claim 1, characterized in that formaldehyde or formaldehyde-affording compounds are reacted in the presence of mesoionic catalysts of the formula I 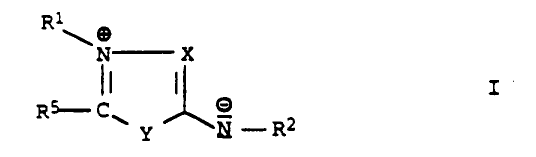
where X, Y and R⁵ are each as defined in claim 1 and where
the radicals R¹, R and R³ are identical or different and represent C₁- to C₃₀-alkyl groups, C₂- to C₃₀-alkenyl or -alkynyl groups having 1 or 2 multiple bonds, C₃- to C₂₀-cycloalkyl or -cycloalkenyl groups, C₃- to C₂₀-heterocycloalkyl or -cycloalkenyl groups, carbon-attached C₂to C₃₀-alkoxy groups having one or more oxygen atoms in the ether chain, C₁- to C₃₀fluorine-, chlorine- and/or bromine-containing haloalkyl groups having one or more halogen atoms, carbon-attached C₂- to C₃₀-secondary amino groups or C₃- to C₃₀-tertiary amino groups, optionally substituted C₆- to C₁₄-aryl groups, optionally substituted C₇- to C₂₀-aralkyl groups and/or optionally substituted C₂- to C₁₅-hetaryl groups having 1 to 3 nitrogen atoms or an oxygen or sulfur atom or having 1 to 2 nitrogen atoms and an oxygen or sulfur atom in the ring, and
the radical R⁴ is identical to or different from the radicals R¹, R or R³ or combines with the radical R to form a C₂- to C₅alkylene or alkenylene or a C₆- to C₁₄-arylene bridge.

3. A process as set forth in claim 1, characterized in that the catalyst used is a mesoionic triazolium compound of the formula Ia 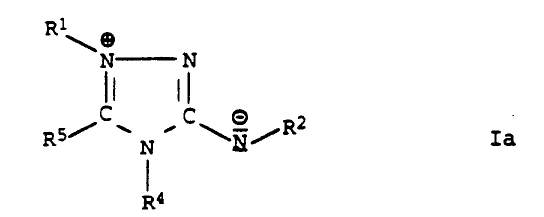 where R¹, R, R⁵ and R⁴ are each as defined in claim 1.

4. A process as set forth in claim 1, characterized in that the catalyst used is nitron, which has the formula Ib

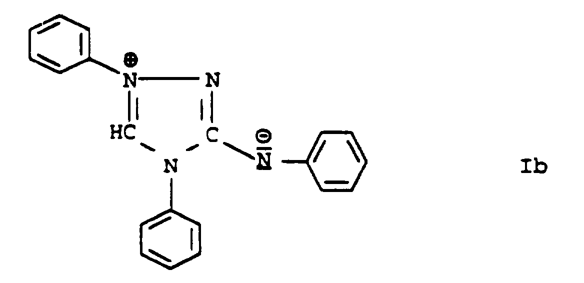

5. A process as set forth in claim 3, characterized in that the catalysts of the formula Ia wherein R⁵ is hydrogen are generated in situ in the reaction mixture from aminoguanidine compounds of the formula II 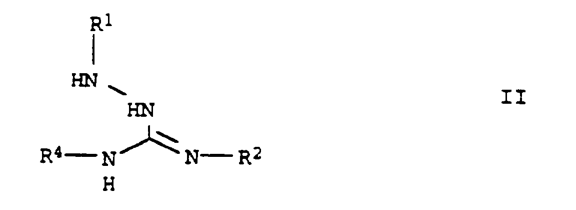 where R¹, R and R⁴ are each as defined in claim 1, and formic acid or from the aminoguanidine compounds II and formaldehyde in the presence of an oxidizing agent.

6. A process as claimed in claim 1, characterized in that the mesoionic catalysts I are used in the form of their onium salts with carboxylic acids or mineral acids and the catalysts I are liberated therefrom with the aid of a base.

## Revendications

1. Procédé de préparation catalytique de produits de condensation du formaldéhyde, caractérisé en ce que l'on fait réagir le formaldéhyde ou des composés qui engendrent du formaldéhyde, en présence de catalyseurs mésoioniques de la formule I 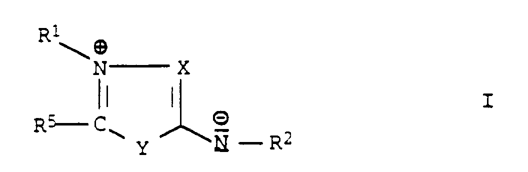 dans laquelle
X représente un atome d'azote ou un radical CR³,
Y représente un atome de soufre ou de sélénium, ou un radical NR⁴ et
les symboles R¹, R et R³ ont des significations identiques ou différentes et représentent des radicaux aliphatiques qui comportent de 1 à 30 atomes de carbone, des radicaux aryle éventuellement substitués, des radicaux aralkyle éventuellement substitués et/ou des radicaux hétéroaryle éventuellement substitués,
le symbole R⁴ représente un radical aliphatique qui comporte de 1 à 30 atomes de carbone, un radical aryle éventuellement substitué, un radical aralkyle éventuellement substitué, ou un radical hétéroaryle éventuellement substitué, ou bien
le symbole R représente en commun avec le symbole R⁴ un radical alcénylène ou alkylène en C₂ à C₅, ou un pont arylène en C₆ à C₁₄ et
le symbole R⁵ représente un atome d'hydrogène, le radical hydroxyméthylène -CH₂OH, ou le radical hydroxyhydroxyméthylèneméthylidyne -CH(OH) (CH₂OH).

2. Procédé suivant la revendication 1, caractérisé en ce que l'on fait réagir le formaldéhyde ou des composés qui engendrent du formaldéhyde en présence de catalyseurs mésoioniques de la formule I 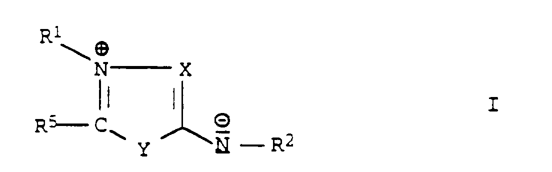 dans laquelle X, Y et R⁵ possèdent les mêmes significations que celles qui leur ont été attribuées dans la revendication 1 et
les symboles R¹, R et R³, qui ont des significations identiques ou différentes, représentent des radicaux alkyle en C₁ à C₃₀, alcényle ou alcynyle en C₂ à C₃₀, comportant 1 ou 2 liaisons multiples, cycloalkyle ou cycloalcényle en C₃ à C₂₀, hétérocycloalkyle ou hétérocycloalcényle en C₃ à C₂₀, des radicaux alcoxy en C₂ à C₃₀ liés par l'intermédiaire d'un atome de carbone au corps de base du catalyseur I, avec un ou plusieurs atomes d'oxygène dans la chaîne éther, des radicaux halogénalkyle contenant du fluor, du chlore et/ou du brome, en C₁ à C₃₀, comportant un ou plusieurs atomes d'halogènes, des radicaux amino tertiaires en C₃ à C₃₀ ou des radicaux amino secondaires en C₂ à C₃₀, liés par l'intermédiaire d'un atome de carbone au corps de base du catalyseur I, des radicaux aryle en C₆ à C₁₄ éventuellement substitués, des radicaux aralkyle en C₇ à C₂₀ éventuellement substitués et/ou des radicaux hétéroaryle en C₂ à C₁₅ éventuellement substitués, comportant 1 à 3 atomes d'azote ou un atome d'oxygène ou de soufre, ou comportant 1 ou 2 atomes d'azote et un atome d'oxygène ou de soufre dans le noyau ou cycle et
le symbole R⁴ a des significations identiques ou différentes de celles des symboles R¹, R ou R³, ou bien forme en commun avec le symbole R un pont alcénylène ou alkylène en C₂ à C₅, ou un pont arylène en C₆ à C₁₄.

3. Procédé suivant la revendication 1, caractérisé en ce que l'on utilise, à titre de catalyseur, un composé de triazolium mésoionique de la formule Ia 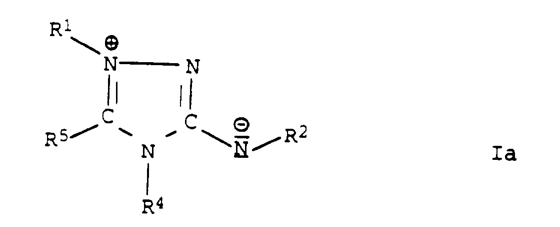 dans laquelle les symboles R¹, R, R⁵ et R⁴ possèdent les mêmes significations que celles qui leur ont été attribuées dans la revendication 1.

4. Procédé suivant la revendication 1, caractérisé en ce que l'on utilise, à titre de catalyseur, le nitron de la formule Ib

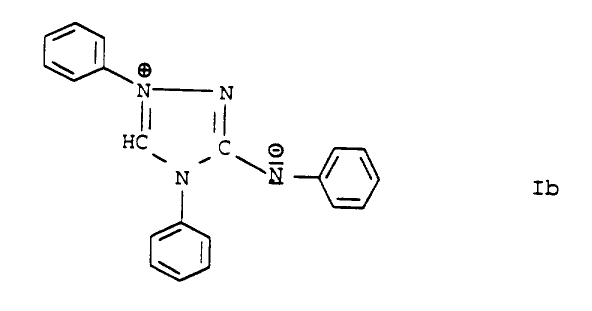

5. Procédé suivant la revendication 3, caractérisé en ce que l'on réalise les catalyseurs de la formule Ia dans laquelle R⁵ représente un atome d'hydrogène, in situ dans le mélange réactionnel, au départ de composés d'aminoguanidine de la formule II 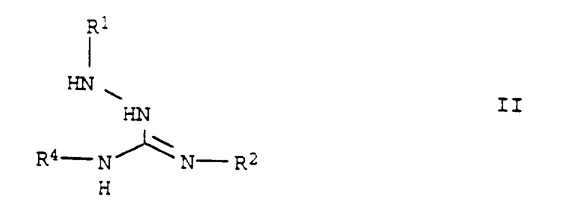 dans laquelle R¹, R et R⁴ possèdent les mêmes significations que celles qui leur ont été attribuées dans la revendication 1 et d'acide formique, ou à partir de composés d'aminoguanidine II et de formaldéhyde, en présence d'un agent d'oxydation.

6. Procédé suivant la revendication 1, caractérisé en ce que l'on met en oeuvre les catalyseurs mésoioniques I sous la forme de sels d'onium avec des acides carboxyliques ou des acides minéraux et on en libère les catalyseurs I à l'aide d'une base.
